# EUROPEAN PATENT APPLICATION

(11) **EP 1 564 289 A1**
(43) Date of publication of application: **17.08.2005**
(21) Application number: 04002923.3
(22) Date of filing: 10.02.2004
(51) Int. Cl.: C12N 15/10, C12P 19/34

(54) **Stabilization of linear double-stranded DNA in the presence of exonucleases**

(71) Applicant: Roche Diagnostics GmbH, 68305 Mannheim (DE); F. Hoffmann-La Roche AG, 4070 Basel (CH)
(72) Inventor: Besir, Hueseyin Dr., 81476 München (DE)

(57) **Abstract**

The present invention is directed at stabilizing linear polynucleotides against exonucleolytic attack. Adding to both ends of a double-stranded linear DNA a target sequence for a DNA-binding protein the double-stranded linear DNA with the two added target sequences has an increased stability against exonucleolytic attack when contacted with the DNA-binding protein.

## Description

The present invention is directed to a method to increase the stability of double-stranded linear polynucleotides in the presence of exonucleases. In addition, the invention provides double-stranded linear polynucleotides stabilized against exonucleolytic attack, as well as compositions containing such stabilized double-stranded linear polynucleotides.

Exonucleases are enzymes of the hydrolase class that catalyze the hydrolysis of terminal bonds of deoxyribonucleotide or ribonucleotide chains, releasing mononucleotides. Exonucleolytic activity is found in a number of DNA (desoxyribonucleicacid) polymerases which are required to maintain the integrity of the genome during processes such as DNA replication, DNA repair, translesion DNA synthesis, DNA recombination, cell-cycle control and DNA-damage-checkpoint function. Many polymerases (for instance, pols γ, δ and ε) contain a 3'-5' exonuclease enzymatic activity in the DNA polymerase polypeptide. This enzymatic activity can proofread for the polymerase, and enhances the accuracy of its DNA synthesis by excising incorrectly polymerized nucleotides. In addition, further autonomous 3'-5' exonucleases are known as well as 5'-3' exonucleases (Shevelev, I.V. and Hübscher, U., Nature Reviews Molecular Cell Biology (2002) 3: 364-376). Exonucleases additionally differ with respect to their specificity. Thus, some exonucleases specifically degrade either single-stranded (ss) or double-stranded (ds) DNA, and some exonucleases are capable of degrading both types. Table 1 gives an overview regarding the exonucleases of Escheichia coli (E. coli).

**Table 1**

| **E. coli exonucleases** | | | | |
|---|---|---|---|---|
| *Name* | *Gene* | *Polarity* | *DNA substrate* | *Function* |
| ExoI | sbcB | 3'-5' | ss | Repair, recombination |
| ExoII | polA | 3'-5' | ss | Replication, repair |
| ExoIII | xthA | 3'-5' | ds | Repair |
| ExoIV | | | ss | |
| ExoV | recB, recC, recD | 3'-5' 5'-3' | ss / ds | Repair, recombination |
| ExoVI | polA | 5'-3' | ds | Repair |
| ExoVII | xseA, xseB | 3'-5' 5'-3' | ss | Repair, recombination |
| ExoVIII | recE | 5'-3' | ds | Recombination |
| ExoIX | xni | 3'-5' | ss (ds) | Repair |
| ExoX | yobC | 3'-5' | ss (ds) | Repair |
| ε subunit of PolIII | dnaQ | 3'-5' | ss | Replication |
| PolII | polB | 3'-5' | ss | Replication, repair |
| RecJ | recJ | 5'-3' | ss | Repair, recombination |
| SbcCD | sbcC, sbcD | ? | ds | Recombination |
| RNaseT | mt | 3'-5' | ss | Repair, processing of tRNA |

In E. coli, the major proofreading 3'-5' exonuclease activity has been attributed to the ε subunit of PolIII whereas the major 5'-3' exonuclease activity has been attributed to ExoVII. Remarkably, ExoV has an ATP (adenosinetriphosphate) dependent exonucleolytic activity on ss and ds DNA, and an ATP independent endonucleolytic activity on ss DNA.

### Background art

Degradation of linear DNA molecules is a widely known and frequent problem when applying certain methods of molecular biology. For example, cell-free DNA-dependent in vitro transcription and translation works quite well in practice with respect to the expression of circular double helix DNA and with respect to the expression of long linear DNA. Attempts at expressing shorter linear DNA fragments had only limited success. The smaller the DNA that is used the more difficult it was to obtain appreciable amounts of the gene product. It was established that these difficulties were due to the presence of exonucleases in the cell-free extracts. Hence, it was shown that ExoV is responsible for the degradation of linear DNA when S30 lysates of E. coli were transcribed and translated in vitro.

ExoV (exonuclease V) is composed of three subunits (the gene products of recB, recC, redD). A previous approach to increase the stability of linear DNA molecules in cell-free E. coli lysates was to mutate the genes encoding the ExoV subunits. Yang, H.L. et al., (1980) Proc Natl Acad Sci USA 77:7029-7033 describe an improved protein synthesis starting from linear DNA templates using the E. coli strain CF300 after deletion of ExoV (elimination of the genes recB, recC; strain recB21). Jackson, M. er al., FEBS Lett. (1983) 163:221-224 describe the preparation of an improved in vitro coupled transcription-translation system from E. coli strains lacking ExoV. Bassett, C.L and Rawson, J.R. J Bacteriol (1983) 156:1359-1362 additionally mutated the RNase and polynucleotide phosphorylase genes (rna-19, pnp-7) in the recB21 strain (strain CLB7) and achieved a significantly higher protein expression with linear DNA templates after a one hour incubation period. Lesley, S.A. et al., J Biol Chem (1991) 266:2632-2638 used an ExoV-deficient recD BL21 strain which was referred to as the SL119 strain and describe for the first time the method of in vitro protein synthesis starting from PCR-generated templates. Lysates of the strain SL119 are commercially available (Promega) for in vitro transcription and translation using linear templates.

A shortcoming of the mutational measures described above is that the E. coli mutants mentioned grow more slowly than the wildtype and also the lysates obtained from these strains have a significantly poorer rate of synthesis. Apparently, ExoV plays an important role in the metabolism of the bacteria. Hence, when producing cell-free extracts for coupled transcription and translation in vitro it appears to be important to use lysates in which exonucleases are present.

Another conceivable measure to protect linear DNA against exonucleolytic degradation is to modify the nucleic acids either by covalently attaching protecting grops to the nucleotides at both ends of the linear DNA or by incorporating certain modified nucleotides. Single-stranded DNA or RNA molecules can be protected by protecting the ends with alkyl groups and by modifying the bases (Pandolfi, D. et al., (1999) Nucleosides Nucleotides. 18:2051-2069). Verheijen, J.C. et al. (2000) Bioorg Med Chem Lett 10:801-804 show an increased stability of single-stranded DNA molecules by protecting the ends with 4-hydroxy-N-acetylprolinol, L-serinol or by 3'-3'-phosphodiester bonds. Kandimalla, E.R. et al., Nucleic Acids Res (1997) 25:370-378 describe mixed backbone oligonucleotides containing 2'-5'-ribo- and 3'-5'-deoxyribonucleotide segments. Pure or mixed phosphorothioate bonds and chemically modified oligonucleotides e.g. methylphosphonates and phosphoramidates are more stable and are degraded more slowly by exonucleases. Tohda, H. et al., J Biotechnol (1994) 34:61-69 show that RNA containing phosphorothioates is more stable towards nucleases and therefore has a higher translation efficiency. However, on the whole only small amounts of protein could be produced. Tang, J.Y. et al., Nucleic Acids Res (1993) 21:2729-2735 describe oligonucleotides that have hairpin loop structures at their 3' ends, and show increased resistance to degradation by exonucleases. Hirao, I. et al., FEBS Lett (1993) 321:169-172 show that the hairpin, which the DNA fragment d(GCGAAGC) forms, is extremely resistant to nucleases from E. coli extracts. Yoshizawa, S. et al., Nucleic Acids Res (1994) 22:2217-2221 describe that a stabilization of the 3' end of mRNA by hybridization with the same hairpin results in a 200-fold increase in the efficiency of in vitro translation with E. coli extracts. Good, L and Nielsen, P.E. Proc Natl Acad Sci USA (1998) 95:2073-2076 show that synthetic molecules containing bases that are coupled to a peptide backbone (peptide nucleic acid, PNA) are resistant to hydrolytic cleavage in E. coli extracts and can be used as anti-sense molecules. Burdick, G. and Emlen, W. J Immunol (1985) 135:2593-2597 describe that in DNA anti-DNA immunocomplexes bound IgG molecules can protect DNA from nucleolytic degradation.

WO 90/15065 discloses oligonucleotides with phosphoramidite linkages at the 3' end and the 5' end. WO 94/16090 discloses the use of exonuclease-resistant single-stranded DNA oligonucleotides containing nucleotide derivatives in which one or two of the non-bridging oxygens in the phosphate moiety of a nucleotide has been replaced with a sulfur-containing group (e.g. a phosphothioate), an alkyl group (e.g. a methyl or ethyl group), a nitrogen-containing group (e.g. an amine), or a selenium-containing group. WO 00/40592 discloses exonuclease-resistant nucleic acids modified by replacing at the 3' end the hydrogen atom at the 3' or the 2' position on the sugar molecule by a chemical moiety such as a butyl or a butanol. In addition, butyl blocking groups are disclosed for the 5' end. WO 03/072051 discloses fluorescence energy transfer labeled oligonucleotides that include a 3'-5' exonuclease resistant quencher domain. EP 0 431 523 discloses a nuclease resistant nucleotide compound which contains an intercalating agent, a methylthiophosphate, a carbodiimide, an N-hydroxybenzotriazole, isourea, a polypeptide or a protein. EP 0 967 274 describes methods for preparing dumbbell-shaped linear double-stranded DNA molecules with increased stability towards exonucleolytic activity. US 5,245,022 dicloses nuclease resistant oligonucleotides with a diol such as tetraethylene glycol at either or both termini.

Cell-free in vitro expression systems but without strategies for protection of linear template DNA against exonucleolytic attack are described in the prior art. In US 5,571,690 Hecht describes a method for the cell-free synthesis of a protein starting with a template which was generated in a PCR reaction. In this method the entire gene sequence including the phage promoter region is amplified from a plasmid. After in vitro transcription a lysate from rabbit reticulocytes is used for translation. With this method it was possible to produce 57 µg/ml of a protein using mRNA which was modified after transcription with a 5'CAP. Martemyanov, K.A. et al., FEBS Lett (1997) 414:268-270 use an S30 extract from E. coli for the cell-free synthesis of a protein starting with a template which was generated in a 2-step PCR reaction. In this method the target gene is firstly amplified in a PCR reaction with the aid of two gene-specific oligonucleotide primers and subsequently subjected to a second PCR reaction in which a so-called megaprimer is used to fuse the T7 promoter and the ribosomal binding site to the amplified gene. It was however only possible to produce radioactively detectable amounts of protein. Yang, J. et al., J Bacteriol (2000) 182:295-302 use an S30 extract from E. coli to demonstrate the cell-free synthesis of a protein starting with a template which was generated in a PCR reaction. It was only possible in this method to produce radioactively detectable amounts of protein. Nakano, H. et al., (1999) Biotechnol Bioeng 64:194-199 use an S30 extract from E. coli in a hollow fibre reactor to at least produce 80 µg/ml protein reaction mixture starting with a template which was generated in a PCR reaction. US 6,027,913 discloses an extract from reticulocytes for the cell-free synthesis of a protein starting with a template which was generated in a single step PCR reaction. In this method the T7 promoter and the ribosomal binding site are fused to the target gene. Even with this method only small amounts of protein were produced.

Although eukaryotic lysates from rabbit reticulocytes are relatively nuclease-free, it is a shortcoming that these lysates cannot be produced economically in large amounts. They only allow very small protein yields. The same applies to lysates from wheat germs which either have prepared in a laborious process or they otherwise may be strongly contaminated with translation-inhibiting factors.

In contrast, E. coli lysates yield much larger amounts of protein. However, the described methods for preparing lysates from E. coli only allow relatively short reaction periods of up to about one hour with linear DNA templates since afterwards these DNA templates are completely degraded by the exonucleases contained in the lysate. The lysates obtained from E. coli exonuclease mutants (i.e. exonuclease-deficient strains) have a significantly poorer synthesis performance than comparable wildtype strains such as, e.g. the A19 strain. Methods for protecting mRNA have the disadvantage that firstly an in vitro transcription has to be carried out before the protected mRNA can be added to the lysate. This in turn does not permit a coupled reaction and a continuous RNA synthesis. Methods for protecting RNA are described in Tohda, H. et al., J Biotechnol (1994) 34:61-69, Yoshizawa, S. et al., Nucleic Acids Res(1994) 22:2217-2221.

EP 1 251 168 discloses methods for improving the stability of linear DNA in cell-free in vitro transcription and translation systems. The document discloses that the yield of protein expressed in the cell-free expression system can be increased by adding unspecific linear DNA to the exonuclease-containing lysates.

It is also known to the skilled artisan that DNA-binding proteins or DNA-binding protein complexes protect the DNA to which they bind to. Wu, C. Nature (1985) 317:84-87 describes an exonuclease protection assay on free linear DNA for detecting sequence-specific DNA-binding proteins. The assay method was used to fractionate from a crude extract the protein factor that is bound constitutively to a heat-shock gene TATA box region in native chromatin of Drosophila cells. However, the protection assay in the cited document (as well as numerous other DNAse protection assays that are documented in the literature) was aimed at locating the boundaries of protein-binding on DNA.

The the methods of the state of the art have certain disadvantages. Therefore, the problem solved by the present invention is to provide an alternative method for producing a double-stranded polynucleotide, that is to say a double-stranded DNA, that is stabilized against exonucleolytic attack. The inventors have unexpectedly found that adding to both ends of the double-stranded linear DNA a target sequence for a DNA-binding protein the double-stranded linear DNA with the two added target sequences has an increased stability against exonucleolytic attack when contacted with the DNA-binding protein.

As discussed above, such a stabilized double-stranded DNA (polynucleotide) presents a particular advantage when used as a template DNA in cell-free expression systems, that is to say in a cell-free reaction mixture for coupled transcription and translation. However, further advantageous applications of a stabilized double-stranded polynucleotide according to the invention are possible.

### Summary of the invention

Therefore, a first aspect of the invention is a method to produce a double-stranded linear polynucleotide stabilized against exonucleolytic attack, comprising the steps of (i) providing a single- or double-stranded polynucleotide; (ii) providing a first single-stranded oligonucleotide consisting of (a) a 3'-terminal nucleotide sequence of 7 to 25 nucleotides complementary to the nucleotide sequence at the 3'-end of the polynucleotide, (b) a first operator adjacent to (a), (c) a first 5'-terminal nucleotide sequence adjacent to (b) and consisting of 1 to 15 nucleotides, and a second single-stranded oligonucleotide consisting of (d) a 3'-terminal nucleotide sequence of 7 to 25 nucleotides identical to the nucleotide sequence at the 5'-end of the polynucleotide, (e) a second operator adjacent to (d), (f) a second 5'-terminal nucleotide sequence adjacent to (e) and consisting of 1 to 15 nucleotides; (iii) providing an amplification reaction mixture and adding to the amplification reaction mixture the polynucleotide and the first and the second single-stranded oligonucleotide primer; (iv) annealing in the resulting mixture of step (iii) the first and/or the second single-stranded oligonucleotide to the polynucleotide; (v) amplifying the polynucleotide by means of the polymerase chain reaction thereby producing a double-stranded linear polynucleotide; (vi) providing a first agent capable of binding to the operator of step (ii) (b) and a second agent capable of binding to the operator of step (ii) (e) and contacting the double-stranded linear polynucleotide of step (v) with both, the first agent capable of binding to the operator of step (ii) (b) and the second agent capable of binding to the operator of step (ii) (e), whereby each of the first and the second agent capable of binding to an operator is selected from the group consisting of (A) a DNA-binding protein, (B) a DNA-binding protein complex, (C) a DNA-binding protein or a DNA-binding protein complex and a cofactor binding to the DNA-binding protein or the DNA-binding protein complex, thereby producing a double-stranded linear polynucleotide stabilized against exonucleolytic attack. Another aspect of the present invention is a double-stranded linear polynucleotide stabilized against exonucleolytic attack, obtainable by the method according to the invention and mentioned above. Another aspect of the present invention is a composition comprising an aequous solution containing an exonuclease and a double-stranded linear polynucleotide stabilized against exonucleolytic attack, obtainable by the method according to the invention and mentioned above.

### Detailed description

As used herein, the term "repressor" refers to molecules which act to inhibit the expression of a target structural gene by binding to a specific operator functionally linked to the target structural gene, and the term "repressor gene" refers to a gene which codes for the repressor molecule. The term "operator" as used herein refers to a specific site on a nucleic acid at which a repressor specifically binds, such specific binding blocking the expression of an associated structural gene. Typically, repressors act by preventing the transcription of a structural gene into mRNA by blocking the attachment of RNA polymerase to the nucleic acid. Therefore, when repressor molecules are not present, RNA polymerase can bind to nucleic acid and initiate transcription, and, when repressor molecules are present, the RNA polymerase is prevented from binding to the nucleic acid, and transcription, and therefore expression, is effectively blocked. According to the invention, a preferred repressor is selected from the group consisting of (A) a DNA-binding protein, (B) a DNA-binding protein complex, and (C) a DNA-binding protein or a DNA-binding protein complex and a cofactor binding to the DNA-binding protein or the DNA-binding protein complex. Binding of the repressor to the operator may be increased when a cofactor binds to the repressor. Such a cofactor is known to the person skilled in the art as a corepressor or an anti-inducer. An example therefor is orthonitrophenylfucoside binding to the E. coli Lac repressor (Bell, C.E. and Lewis, M. Curr Opin Struct Biol (2001) 11:19-25). Another example is hypoxanthine binding to the E. coli purine repressor. A repressor may be bound by more than one cofactor. An example therefor is hypoxanthine and guanine binding to the E. coli purine repressor (Choi, K.Y. and Zalkin, H. J Bacteriol (1992) 174:6207-6214).

It is understood that the term "repressor" also comprises a variant, by way of amino acid addition, deletion or exchange, of the repressor. In case the repressor does not consist of a single DNA-binding protein but is a DNA-binding protein complex, the variant therof contains an amino acid addition, deletion or exchange in at least one subunit of the complex. It is also understood that the variant remains capable of operator binding.

Similarly, the term "operator" comprises a variant, by way of nucleotide addition, deletion or exchange, of the operator. It is understood that the variation is such that the binding of the repressor to the operator remains possible.

As used herein, the term "oligonucleotide" is used for a DNA molecule, with up to 100 nucleotides in length. The term "polynucleotide" is used for a DNA molecule with more than 100 nucleotides in length.

The terms "to amplify", "amplifying" and "amplification" are understood to imply the use of the polymerase chain reaction (PCR). PCR is an in vitro method for producing large amounts of specific DNA or RNA fragments of defined length and sequence from small amounts of short oligonucleotide flanking sequences (primers). The essential steps include thermal denaturation of the double-stranded target molecules, annealing of the primers to their complementary sequences, and extension of the annealed primers by enzymatic synthesis with DNA polymerase. Methods to perform PCR in order to amplify a nucleic acid as well as amplification reaction mixtures are well known to the person skilled in the art and include the teachings of US 4,683,195 as well as various other publications such as Sambrook, Fritsch & Maniatis, Molecular Cloning, A Laboratory Manual, 3rd edition, CSHL Press, 2001.

A first aspect of the present invention is a method to produce a double-stranded linear polynucleotide stabilized against exonucleolytic attack, comprising the steps of (i) providing a single- or double-stranded polynucleotide; (ii) providing a first single-stranded oligonucleotide consisting of (a) a 3'-terminal nucleotide sequence of 7 to 25 nucleotides complementary to the nucleotide sequence at the 3'-end of the polynucleotide, (b) a first operator adjacent to (a), (c) a first 5'-terminal nucleotide sequence adjacent to (b) and consisting of 1 to 15 nucleotides, and a second single-stranded oligonucleotide consisting of (d) a 3'-terminal nucleotide sequence of 7 to 25 nucleotides identical to the nucleotide sequence at the 5'-end of the polynucleotide, (e) a second operator adjacent to (d), (f) a second 5'-terminal nucleotide sequence adjacent to (e) and consisting of 1 to 15 nucleotides; (iii) providing an amplification reaction mixture and adding to the amplification reaction mixture the polynucleotide and the first and the second single-stranded oligonucleotide primer; (iv) annealing in the resulting mixture of step (iii) the first and/or the second single-stranded oligonucleotide to the polynucleotide; (v) amplifying the polynucleotide by means of the polymerase chain reaction thereby producing a double-stranded linear polynucleotide; (vi) providing a first agent capable of binding to the operator of step (ii) (b) and a second agent capable of binding to the operator of step (ii) (e) and contacting the double-stranded linear polynucleotide of step (v) with both, the first agent capable of binding to the operator of step (ii) (b) and the second agent capable of binding to the operator of step (ii) (e), whereby each of the first and the second agent capable of binding to an operator is selected from the group consisting of (A) a DNA-binding protein, (B) a DNA-binding protein complex, (C) a DNA-binding protein or a DNA-binding protein complex and a cofactor binding to the DNA-binding protein or the DNA-binding protein complex, thereby producing a double-stranded linear polynucleotide stabilized against exonucleolytic attack.

Thus, the first and the second oligonucleotide represent a pair of primers which are used to amplify the polynucleotide. Essentially, by means of the amplification both termini of the polynucleotide are extended by the addition of an operator sequence and a further terminal sequence (comprising 1 to 15 nucleotides) to each end. Selective hybridization of the two oligonucleotides to the polynucleotide in the course of the PCR process is mediated by the 3'-terminal nucleotide sequences of (a) and/or (d) in step (ii). That is to say, the 3'-terminal nucleotide sequences of (a) and/or (d) in step (ii) hybridize to the polynucleotide. When annealing for the first time the remaining portions of any oligonucleotide, i.e. the operator and the 5'-terminal nucleotide sequence will not hybridize. In the case amplification starts with a single-stranded polynucleotide, following completion of the second PCR cycle, the remaining portions of both oligonucleotides will form the termini of the extended template.

A well-known example for binding of a repressor to an operator is found in the Lac operon of E. coli. The Lac operon encodes in three consecutive reading frames three proteins involved in the metabolism of lactose (glucose-galactose disaccharide): β-galactosidase (also called LacZ) involved in hydrolyzing lactose to form glucose and galactose; a permease that allows lactose to enter cell; and a transacetylase that modifies lactose-like compounds. The Lac operon mRNA synthesis is controlled by a promoter domain at the 5' end. In the situation where no lactose is present, transcription of the operon is blocked by the Lac repressor, also known as LacI, a 37-kDa protein with high affinity for a specific DNA sequence, that is to say the Lac operator present in the promoter. The Lac repressor is synthesized by the LacI gene present elsewhere in the E. coli genome. The operator site of the lac operon has been extensively studied. The nucleotide sequence of the operator site shows a nearly perfect inverted repeat, indicating that the DNA in this region has an approximate twofold axis of symmetry. Symmetry in the operator site usually corresponds to symmetry in the repressor protein that binds the operator site.

In the E. coli cell the lac repressor protein in the absence of lactose binds to the operator and blocks transcription. The lac repressor can exist as a dimer of 37 kDa subunits, and two dimers often come together to form a tetramer. In the absence of lactose, the repressor binds very tightly and rapidly to the operator. When the lac repressor is bound to DNA, it prevents bound RNA polymerase from locally unwinding the DNA to expose the bases that will act as the template for the synthesis of the RNA strand. The lac repressor binds 4 × 10⁶ times as strongly to operator DNA as it does to random sites in the genome. This high degree of selectivity allows the repressor to find the operator efficiently even with a large excess (4.6 x 10⁶) of other sites within the E. coli genome. The dissociation constant for the repressor-operator complex is approximately 0.1 pM (10⁻¹³ M). The rate constant for association (10¹⁰ M⁻¹ s⁻¹) is strikingly high, indicating that the repressor finds the operator by diffusing along a DNA molecule corresponding to a one-dimensional search rather than encountering it from the aqueous medium corresponding to a three-dimensional search.

Inspection of the complete E. coli genome sequence reveals two sites within 500 bp of the primary operator site that approximate the sequence of the operator. Other lac repressor dimers can bind to these sites, particularly when aided by cooperative interactions with the lac repressor dimer at the primary operator site. No other sites that closely match sequence of the lac operator site are present in the rest of the E. coli genome sequence. Thus, the DNA-binding specificity of the lac repressor is sufficient to specify a nearly unique site within the E. coli genome.

The three-dimensional structure of the lac repressor has been determined in various forms. Each monomer consists of a small amino-terminal domain that binds DNA and a larger domain that mediates the formation of the dimer and the tetramer. A pair of the amino-terminal domains come together to form the functional DNA-binding unit. Complexes between the lac repressor and oligonucleotides that contain the lac operator sequence have been structurally characterized. The lac repressor binds DNA by inserting a helix into the major groove of DNA and making a series of contacts with the edges of the base pairs as well as with the phosphodiester backbone. For example, an arginine residue in a helix forms a pair of hydrogen bonds with a guanine residue within the operator. Other bases are not directly contacted but may still be important for binding by virtue of their effects on local DNA structure. As expected, the twofold axis of the operator coincides with a twofold axis that relates the two DNA-binding domains.

Ligand binding can induce structural changes in regulatory proteins such as the E. coli lac repressor. Lactose itself does not have this effect; rather, allolactose, a combination of galactose and glucose with an α-1,6 rather than an α-1,4 linkage, does. Allolactose is thus referred to as the inducer of the lac operon. Allolactose is a side product of the β-galactosidase reaction produced at low levels by the few molecules of β-galactosidase that are present before induction. Some other β-galactosides such as isopropylthiogalactoside (IPTG) are potent inducers of β-galactosidase expression, although they are not substrates of the enzyme.

When the inducer has bound to the lac repressor, the repressor's affinity for operator DNA is greatly reduced. The inducer binds in the center of the large domain within each monomer. This binding leads to local conformational changes that are transmitted to the interface with the DNA-binding domains. The relation between the two small DNA-binding domains is modified so that they cannot easily contact DNA simultaneously, leading to a dramatic reduction in DNA-binding affinity (Berg, J., Tymoczko, J, Stryer, L. (eds.) Biochemistry, 5^{th} ed. W. H. Freeman and Company, New York). As a consequence, the Lac repressor/lactose complex more frequently dissociates from the operator leaving the promoter accessible for RNA polymerase thereby allowing an increased level of transcription of mRNA.

Conversely, an anti-inducer such as, for example, orthonitrophenylfucoside (ONPF) allosterically stabilizes the Lac repressor Lac operator complex by increasing the binding affinity of the Lac repressor to the Lac operator (Choi, K.Y. and Zalkin, H. J Bacteriol (1992) 174:6207-6214; Bell, C.E. and Lewis, M. Curr Opin Struct Biol (2001) 11:19-25).

The operator binding conformation has been shown be stabilized in a variant of the Lac repressor. In the variant the alanine residue at position 110 of the LacI polypeptide has been replaced by a lysine residue (Müller-Hartmann, H. and Müller-Hill, B. J Mol Biol (1996) 257:473-478).

Other combinations of repressors and cognate operators from prokaryotic organisms, including bacteriophages are known to the skilled artisan. Some of the best studied repressor operator complexes are listed in Kercher, M.A., et al. Curr Opin Struct Biol (1997) 7:76-85. Thus, in another preferred embodiment of the invention in step (ii) at least one of the operators of (b) and (e) is a naturally-occurring operator of prokaryotic origin. By way of example, SEQ ID NO:1, SEQ ID NO:2 and SEQ ID NO:3 illustrate naturally-occurring Lac operators from E. coli. SEQ ID NO:4 shows an artificial and symmetrical Lac operator. SEQ ID NO:5 and SEQ ID NO:6 show examples of sequences of the E. coli operator of the purine operon. Thus, in another preferred embodiment of the invention in step (ii) at least one of the operators of (b) and (e) is a variant, by way of addition, deletion or exchange of at least one nucleotide, of the respective naturally-occurring operator of prokaryotic origin.

Furthermore, in another preferred embodiment of the invention in step (vi) each of the first and the second agent capable of binding to the operator is selected from the group consisting of (A) a prokaryotic repressor capable of binding at least one of the operators of (b) and (e) of step (ii); (B) a prokaryotic repressor bound by a corepressor or an anti-inducer, whereby the prokaryotic repressor bound by the corepressor or the anti-inducer is capable of binding at least one of the operators of (b) and (e) of step (ii).

In another preferred embodiment of the invention in step (ii) the operators of (b) and (e) are identical. In yet another preferred embodiment of the invention in step (vi) the first and the second agent capable of binding to the operator are identical.

In another preferred embodiment of the invention in step (ii) at least one operator and in step (vi) at least one agent capable of binding to the operator are selected from the group consisting of the Escherichia coli purine operator according to SEQ ID NO:5 and the Escherichia coli purine repressor with hypoxanthine or, optionally, hypoxanthine and guanine bound to the purine repressor; the Escherichia coli purine operator according to SEQ ID NO:6 and the Escherichia coli purine repressor with hypoxanthine or, optionally, hypoxanthine and guanine bound to the purine repressor.

In another preferred embodiment of the invention in step (ii) at least one operator and in step (vi) at least one agent capable of binding to the operator are selected from the group consisting of (I) the Escherichia coli Lac operator according to SEQ ID NO:1 and the Escherichia coli Lac repressor; (II) the Escherichia coli Lac operator according to SEQ ID NO:1 and the Escherichia coli Lac repressor with orthonitrophenylfucoside binding to the Lac repressor; (III) the variant of the Escherichia coli Lac operator according to SEQ ID NO:4 and the Escherichia coli Lac repressor; (IV) the variant of the Escherichia coli Lac operator according to SEQ ID NO:4 and the Escherichia coli Lac repressor with orthonitrophenylfucoside binding to the Lac repressor.

In another preferred embodiment of the invention in step (ii) at least one operator and in step (vi) at least one agent capable of binding to the operator are the Escherichia coli Lac operator according to SEQ ID NO:1 and a variant of the Escherichia coli Lac repressor, whereby the variant is characterized by an amino acid exchange of the LacI polypeptide in that the alanine residue at position 110 is replaced by a lysine residue. Such a variant is known to be stabilized in the operator binding conformation (Müller-Hartmann, H. and Müller-Hill, B. J Mol Biol (1996) 257:473-478).

Several trans-acting factors, i.e. agents capable of binding to an operator, also exist in eukaryotic organisms. Such agents take part in the transcriptional regulation in yeast in that they that bind to enhancer-like upstream activating sequences (UASs). In the present document "enhancer-like upstream activating sequences" are understood to be encompassed by the term "operator". Thus, in another preferred embodiment of the invention in step (ii) at least one of the operators of (b) and (e) is a naturally-occurring operator of eukaryotic origin or a variant thereof. In an even more preferred embodiment of the invention, the operator is an enhancer-like upstream activating sequence derived from yeast. Even more preferred, the operator is an enhancer-like upstream activating sequence derived from the yeast Saccharomyces cerevisiae. It is also preferred that the operator consists of several copies of the upstream activating sequence of eukaryotic origin.

Particularly, the GCN4 or the GAL4 protein are each considered as exemplifying an eukaryotic agent capable of binding to an operator. GCN4 activates the transcription of many genes involved in amino acid biosynthetic pathways. In response to amino acid starvation, GCN4 protein levels rise and, in turn, increase the levels of the amino acid biosynthetic genes. The UASs recognized by GCN4 contain the principal recognition sequence element ATGACTCAT. Therefore, it is even more preferred that in step (ii) at least one operator consists of one to four copies of the sequence ATGACTCAT and in step (vi) at least one agent capable of binding to the operator is GCN4. It is even more preferred that in the operator the copies of the GCN4 recognition sequence are each joined by a spacer sequence.

GAL4 is involved in activating UASs for the promoters of genes involved in galactose metabolism. GAL4 binds as a dimer to four sites with similar symmetrical 17 bp sequences within these promoters. Liang, S.D., et al. Mol Cell Biol (1996) 16:3773-3780 describe wildtype as well as artificially altered GAL4 recognition sequences with increased GAL4 binding. Thus, in an even more preferred embodiment of the invention in step (ii) at least one operator consists of one or two copies of a sequence selected from the group consisting of SEQ ID NO:7, SEQ ID NO:8, SEQ ID NO:9, SEQ ID NO:10, and SEQ ID NO:11, and in step (vi) at least one agent capable of binding to an operator is GAL4. It is even more preferred that in case the operator contains two copies of a GAL4 recognition sequence, said copies are joined by a spacer sequence.

Another aspect of the invention is a double-stranded linear polynucleotide stabilized against exonucleolytic attack, obtainable by the method of the invention and detailed above. In a preferred embodiment of the invention the polynucleotide encodes a polypeptide. It is very much preferred that the polynucleotide encodes a polypeptide in a contiguous open reading frame. It is even more preferred that the polynucleotide additionally contains a ribosome binding site upstream of the open reading frame. Preferred is a ribosome binding site of prokaryotic origin, more preferred an E. coli ribosome binding site. It is even more preferred that the polynucleotide additionally contains a promoter upstream of the open reading frame and the ribosome binding site. Preferably, the promoter upstream of the open reading frame and the ribosome binding site is a prokaryotic promoter. Very much preferred, the promoter is a phage promoter. Also very much preferred is an Escherichia coli promoter.

Yet, another aspect of the invention is a composition comprising an aequous solution containing an exonuclease and a double-stranded linear polynucleotide stabilized against exonucleolytic attack, obtainable by the method of the invention and detailed above. In a preferred embodiment of the invention the aequous solution containing an exonuclease is a cell-free reaction mixture for coupled transcription and translation, whereby the cell-free reaction mixture contains a first agent capable of binding to the operator according to claim 1 step (ii) (b) and a second agent capable of binding to the operator according to claim 1 step (ii) (e). In vitro systems for coupled transcription and translation are known in the state of the art. In a preferred embodiment of the invention the cell-free reaction mixture is an Escherichia coli lysate. In such a case, using in any of the steps (ii) (b) and (ii) (e) an Escherichia coli derived operator is advantageous as the cognate repressor usually is comprised in the extract. Many repressors in the Escherichia coli cell are expressed constitutively such as the Lac repressor. It is therefore very much preferred to select in any of the steps (ii) (b) and (ii) (e) the E. coli Lac operator or a variant thereof.

It is also possible to express or coexpress in the cell-free reaction mixture for coupled transcription and translation the agent capable of binding to the operator to form said agent capable of binding to the operator. Thus, in another embodiment of the invention the cell-free reaction mixture additionally contains a nucleic acid encoding an agent capable of binding to an operator according to claim 1 step (ii) (b) or (e) that is expressed in the cell-free reaction mixture to provide the agent capable of binding to an operator. E.g., if it is desired to increase the concentration of the Lac repressor in a cell-free reaction mixture for coupled transcription and translation that is based on an E. coli extract, the LacI polypeptide can be expressed in the reaction mixture. It is also possible to express in this case a variant of the LacI polypeptide. Very much preferred is expressing a LacI polypeptide with an amino acid exchange at position 110 from alanine to lysine. Such a variant is known to be stabilized in the operator binding conformation (Müller-Hartmann, H. and Müller-Hill, B. J Mol Biol (1996) 257:473-478). By way of another example, if it is desired to provide GAL4 in a cell-free reaction mixture for coupled transcription and translation that is based on an E. coli extract, GAL4 polypeptide can be expressed in the reaction mixture.

Another possibility to provide the said agent capable of binding to the operator is to synthesize said agent independently. E.g., if it is desired to provide GAL4 in a cell-free reaction mixture for coupled transcription and translation that is based on an E. coli extract, GAL4 polypeptide can be expressed and purified independently, and thenbe to the reaction mixture.

In case the agent capable of binding to an operator is an allosteric repressor it is understood that, in order to achieve the desired effect of the invention, the presence of any cofactor that reduces the affinity of the agent to its cognate operator is preferably minimized. Generally, the lysate which is used to prepare the cell-free reaction mixture for coupled transcription and translation is preferably prepared from an E. coli culture that has been grown in the absence of said cofactor. For example, the Lac repressor is selected as a preferred allosteric repressor. The lysate which is used to prepare the cell-free reaction mixture for coupled transcription and translation is then preferably prepared from an E. coli culture that has been grown in the absence of lactose or allolactose.

Conversely, if the binding of the allosteric repressor is enhanced by a corepressor or an anti-inducer, the cell-free reaction mixture for coupled transcription and translation can be prepared from an E: coli culture that has been grown in the presence of said corepressor or anti-inducer or a precursor of said corepressor or anti-inducer, whereby the precursor is converted in the E. coli cell to form said corepressor or anti-inducer. Alternatively, the corepressor or anti-inducer can be added after the extract has been prepared. By way of example, the binding of the Lac repressor to the lac operator is enhanced in the presence of orthonitrophenylfucoside (ONPF), whereby ONPF binds to the Lac Repressor and stabilizes the operator binding conformation of the Lac repressor. Thus, when stabilizing the polynucleotide in a cell-free reaction mixture for coupled transcription and translation that is based on an extract of E. coli cells, by way of binding of the Lac repressor to its cognate operator on the flanks of the polynucleotide, it is preferred that ONPF is added to the reaction mixture.

In addition, enhancement of an allosteric repressor may require more than one corepressor. In such cases it is preferred that a first and a second corepressor are added to the lysate. A preferred allosteric repressor requiring a first and a second corepressor is the purine repressor with hypoxanthine and guanine preferably being added to the lysate in this case.

Yet another embodiment of the invention is a method for increasing the expression of a desired polypeptide in a cell-free reaction mixture for coupled transcription and translation from a linear double-stranded polynucleotide comprising the reading frame encoding the desired polypeptide, a ribosome binding site, and a promoter, comprising the steps of (α) producing from the linear double-stranded polynucleotide a double-stranded linear polynucleotide stabilized against exonucleolytic attack by way of the method of the invention; (β) adding the stabilized double-stranded linear polynucleotide to the cell-free reaction mixture; (γ) incubating the cell-free reaction mixture to form the desired polypeptide.

The following examples, references, sequence listing and figures are provided to aid the understanding of the present invention, the true scope of which is set forth in the appended claims. It is understood that modifications can be made in the procedures set forth without departing from the spirit of the invention.

### Description of the Figures

- **Figure 1**: Linear templates for in vitro transcription/translation of GFP without (NlacO) and with (lacO) flanking lac operator sequence, according to Example 1. The left lane contains size marker fragments.
- **Figure 2**: Yield of GFP as measured by determining GFP fluorescence, according to Example 2.
- **Figure 3**: Yield of GFP as measured by Western blotting, according to Example 3.

### Example 1

### Production of a double-stranded linear DNA

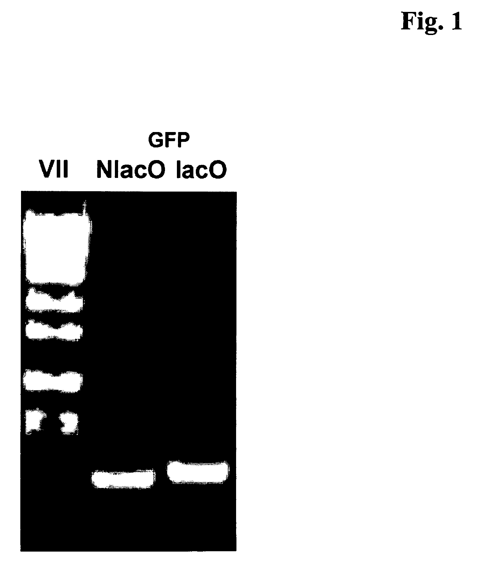

To assess the stabilizing effect of the Lac repressor on a linear DNA molecule containing a Lac operator sequence on both ends of the molecule, a DNA fragment according to SEQ ID NO:12 with the gene for GFP (green fluorescent protein) was amplified from pIVEX2.3GFP (Figure 1) using in separate amplification reactions the two primer pairs lacOs/lacOr (SEQ ID NO:14 and SEQ ID NO:15) and NlacOs/NlacOr (SEQ ID NO:16 and SEQ ID NO:17). The fragment corresponded to the DNA fragment generated by means of the RTS (rapid translation system) Linear Template Generation Set (LTGS, His-tag; Roche Diagnostics GmbH, Mannheim, Cat.No.: 3186237) and contained all necessary elements for in vitro transcription/translation. One fragment was amplified using primers with the same sequence as the standard outer primers from the LTGS plus additional 10 bases at the 5'-end. As a control, a second fragment with a pair of primers containing additional 21 bases of the lac operator downstream of the terminal 6 bases. was generated.

### Example 2

### Double-stranded linear DNA in a cell-free reaction mixture for coupled transcription and translation containing exonucleases

3 µl aliquots of each PCR were incubated in 50 µl of the RTS 100 HY transcription/translation mixture according to the manufacturer's protocol (30°C) both in batch and continuous exchange (CECF) mode. For the CECF reactions 1 ml of the feeding solution from the RTS 500 HY kit was used.

For co-expression of the LacI protein, 3 µg/ml of the vector pIVEXlacI was added to the reaction, while control reactions contained no pIVEXlacI vector (i.e. the LacI coding sequence comprised in SEQ ID NO: 13 inserted into the pIVEX vector). Finally 10 µg/ml of the original expression vector pIVEX2.3GFP was used as control with and without coexpression of LacI.

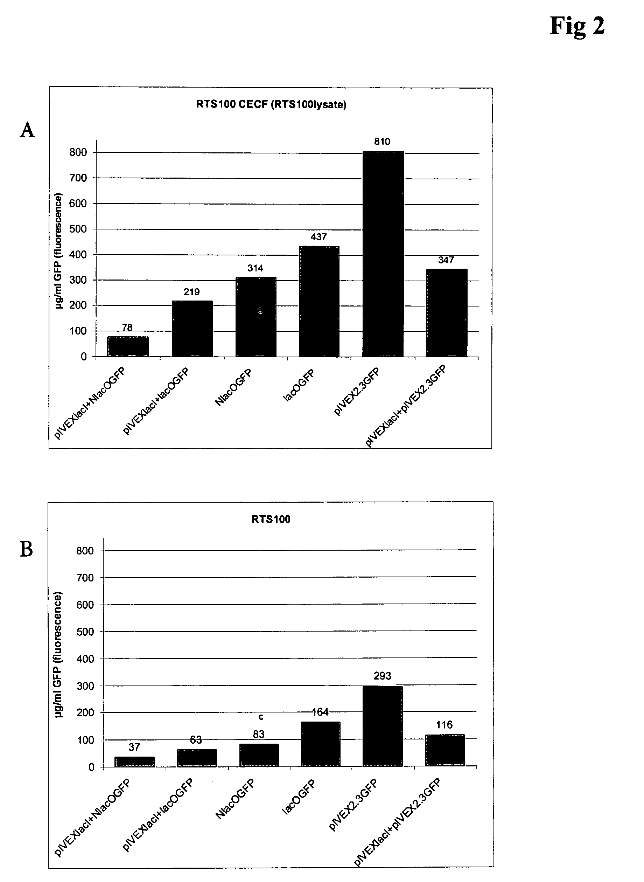

The batch reactions were run for 4 h without shaking, while the CECF reactions were incubated for 6 h with shaking at 900 rpm in the ProteoMaster instrument. After incubation the reactions were kept at 4°C overnight for maturation of the GFP chromophore. Figure 2 shows the resulting quantities of the reactions (A: CECF-mode, B: batch mode).

### Example 3

### Comparison of yield

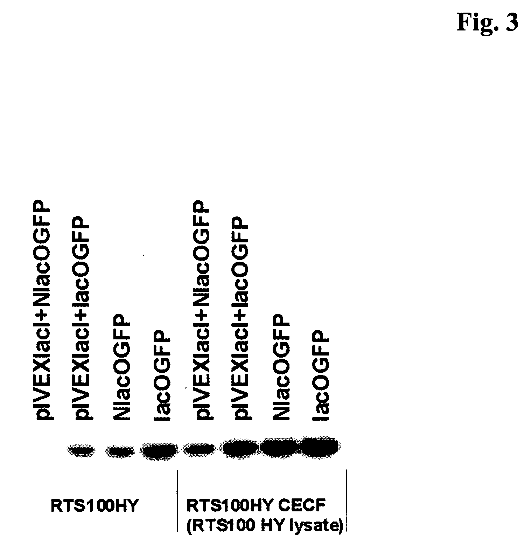

As shown in Figure 3 the differences in yield quantified by measuring GFP fluorescence were confirmed by Western blotting. The presence of the lac operator sequence in the PCR fragments resulted in a significant increase in yield compared to the unprotected fragments, both in batch (left side of Figure 3) and CECF mode (right side of Figure 3). An increase of about 90% in batch and 40% in CECF reactions without co-expression show the stabilizing effect of the LacI protein already present in the lysate. Co-expression leads to lower absolute yields but to higher relative differences between protected and unprotected fragments.

### List of References

Bassett, C.L and Rawson, J.R. J Bacteriol (1983) 156:1359-1362
Bell, C.E. and Lewis, M. Curr Opin Struct Biol (2001) 11:19-25
Berg, J., Tymoczko, J, Stryer, L. (eds.) Biochemistry, 5th ed. W. H. Freeman and Company, New York
Burdick, G. and Emlen, W. J Immunol (1985) 135:2593-2597
Choi, K.Y. and Zalkin, H. J Bacteriol (1992) 174:6207-6214
EP 0 431 523
EP 0 967 274
EP 1 251 168
Good, L and Nielsen, P.E. Proc Natl Acad Sci USA (1998) 95:2073-2076
Hirao, I. et al., FEBS Lett (1993) 321:169-172
Jackson, M. er al., FEBS Lett. (1983) 163:221-224
Kandimalla, E.R. et al., Nucleic Acids Res (1997) 25:370-378
Kercher, M.A., et al. Curr Opin Struct Biol (1997) 7:76-85
Lesley, S.A. et al., J Biol Chem (1991) 266:2632-2638
Liang, S.D., et al. Mol Cell Biol (1996) 16:3773-3780
Martemyanov, K.A. et al., FEBS Lett (1997) 414:268-270
Müller-Hartmann, H. and Müller-Hill, B. J Mol Biol (1996) 257:473-478
Nakano, H. et al., (1999) Biotechnol Bioeng 64:194- 199
Pandolfi, D. et al., (1999) Nucleosides Nucleotides. 18:2051-2069
Sambrook, Fritsch & Maniatis, Molecular Cloning, A Laboratory Manual, 3rd edition, CSHL Press, 2001
Shevelev, I.V. and Hübscher, U., Nature Reviews Molecular Cell Biology (2002) 3: 364-376
Tang, J.Y. et al., Nucleic Acids Res (1993) 21:2729-2735
Tohda, H. et al., J Biotechnol (1994) 34:61-69
Yoshizawa, S. et al., Nucleic Acids Res( 1994) 22:2217-2221
US 4,683,195
US 5,245,022
US 5,571,690
US 6,027,913
Verheijen, J.C. et al. (2000) Bioorg Med Chem Lett 10:801-804
WO 00/40592
WO 03/072051
WO 90/15065
WO 94/16090
Wu, C. Nature (1985) 317:84-87
Yang, H.L. et al., (1980) Proc Natl Acad Sci USA 77:7029-7033
Yang, J. et al., J Bacteriol (2000) 182:295-302
Yoshizawa, S. et al., Nucleic Acids Res (1994) 22:2217-2221

## Claims

1. A method to produce a double-stranded linear polynucleotide stabilized against exonucleolytic attack, comprising the steps of
(i) providing a single- or double-stranded polynucleotide;
(ii) providing a first single-stranded oligonucleotide consisting of
(a) a 3'-terminal nucleotide sequence of 7 to 25 nucleotides complementary to the nucleotide sequence at the 3'-end of the polynucleotide,
(b) a first operator adjacent to (a),
(c) a first 5'-terminal nucleotide sequence adjacent to (b) and consisting of 1 to 15 nucleotides,
and a second single-stranded oligonucleotide consisting of
(d) a 3'-terminal nucleotide sequence of 7 to 25 nucleotides identical to the nucleotide sequence at the 5'-end of the polynucleotide,
(e) a second operator adjacent to (d),
(f) a second 5'-terminal nucleotide sequence adjacent to (e) and consisting of 1 to 15 nucleotides;
(iii) providing an amplification reaction mixture and adding to the amplification reaction mixture the polynucleotide and the first and the second single-stranded oligonucleotide primer;
(iv) annealing in the resulting mixture of step (iii) the first and/or the second single-stranded oligonucleotide to the polynucleotide;
(v) amplifying the polynucleotide by means of the polymerase chain reaction thereby producing a double-stranded linear polynucleotide;
(vi) providing a first agent capable of binding to the operator of step (ii) (b) and a second agent capable of binding to the operator of step (ii) (e) and contacting the double-stranded linear polynucleotide of step (v) with both, the first agent capable of binding to the operator of step (ii) (b) and the second agent capable of binding to the operator of step (ii) (e),
whereby each of the first and the second agent capable of binding to an operator is selected from the group consisting of
(A) a DNA-binding protein,
(B) a DNA-binding protein complex,
(C) a DNA-binding protein or a DNA-binding protein complex and a cofactor binding to the DNA-binding protein or the DNA-binding protein complex,
thereby producing a double-stranded linear polynucleotide stabilized against exonucleolytic attack.

2. The method according to claim 1, **characterized in that**
in step (ii) at least one of the operators of (b) and (e) is a naturally-occurring operator of prokaryotic origin.

3. The method according to any of the claims 1 and 2, **characterized in that**
in step (ii) at least one of the operators of (b) and (e) is a variant, by way of addition, deletion or exchange of at least one nucleotide, of the respective naturally-occurring operator of prokaryotic origin.

4. The method according to any of the claims 1 and 3, **characterized in that**
in step (vi) each of the first and the second agent capable of binding to the operator is selected from the group consisting of
(A) a prokaryotic repressor capable of binding at least one of the operators of (b) and (e) of step (ii);
(B) a prokaryotic repressor bound by a corepressor or an anti-inducer, whereby the prokaryotic repressor bound by the corepressor or the anti-inducer is capable of binding at least one of the operators of (b) and (e) of step (ii).

5. The method according to any of the claims 1 to 4, **characterized in that**
in step (ii) the operators of (b) and (e) are identical.

6. The method according to claim 5, **characterized in that**
in step (vi) the first and the second agent capable of binding to the operator are identical.

7. The method according to any of the claims 1 to 6, **characterized in that**
in step (ii) at least one operator and in step (vi) at least one agent capable of binding to the operator are selected from the group consisting of
(I) the Escherichia coli Lac operator according to SEQ ID NO:1 and the Escherichia coli Lac repressor;
(II) the Escherichia coli Lac operator according to SEQ ID NO:1 and the Escherichia coli Lac repressor with orthonitrophenylfucoside binding to the Lac repressor;
(III) the variant of the Escherichia coli Lac operator according to SEQ ID NO:4 and the Escherichia coli Lac repressor;
(IV) the variant of the Escherichia coli Lac operator according to SEQ ID NO:4 and the Escherichia coli Lac repressor with orthonitrophenylfucoside binding to the Lac repressor.

8. A double-stranded linear polynucleotide stabilized against exonucleolytic attack, obtainable by the method according to any of the claims 1 to 7.

9. A composition comprising an aequous solution containing an exonuclease and a double-stranded linear polynucleotide stabilized against exonucleolytic attack, obtainable by the method according to claim 8.

10. The composition according to claim 9, **characterized in that**
the aequous solution containing an exonuclease is a cell-free reaction mixture for coupled transcription and translation,
whereby the cell-free reaction mixture contains a first agent capable of binding to the operator according to claim 1 step (ii) (b) and a second agent capable of binding to the operator according to claim 1 step (ii) (e).

11. The composition according to claim 10, **characterized in that**
the cell-free reaction mixture additionally contains a nucleic acid encoding an agent capable of binding to an operator according to claim 1 step (ii) (b) or (e) that is expressed in the cell-free reaction mixture to provide the agent capable of binding to an operator.

12. The composition according to any of the claims 10 and 11, **characterized in that**
the cell-free reaction mixture is an Escherichia coli lysate.


SEQUENCE LISTING

<110> Roche Diagnostics GmbH
F. Hoffmann-La Roche AG

<120> Stabilization of linear double-stranded DNA in the presence of
exonucleases

<130> 22369 EP

<160> 17

<170> PatentIn version 3.2

<210> 1
<211> 21
<212> DNA
<213> Escherichia coli

<220>
<221> misc_feature
<222> (1)..(21)
<223> naturally-occurring wildtype Lac operator O1 as described in
Bell, C.E. & Lewis, M., Current Opinion in Structural Biology
(2001) 11:19-25

<400> 1
aattgtgagc ggataacaat t 21

<210> 2
<211> 21
<212> DNA
<213> Escherichia coli

<220>
<221> misc_feature
<222> (1)..(21)
<223> naturally-occurring wildtype Lac operator O2 as described in
Bell, C.E. & Lewis, M., Current Opinion in Structural Biology
(2001) 11:19-25

<400> 2
aaatgtgagc gagtaacaac c 21

<210> 3
<211> 21
<212> DNA
<213> Escherichia coli

<220>
<221>  misc_feature
<222>  (1)..(21)
<223>  naturally-occurring wildtype Lac operator O3 as described in
       Bell, C.E. & Lewis, M., Current Opinion in Structural Biology
       (2001) 11:19-25

<400>  3
ggcagtgagc gcaacgcaat t                                                21


<210>  4
<211>  21
<212>  DNA
<213>  Artificial

<220>
<223>  modified, symmetrical Lac operator


<220>
<221>  misc_feature
<222>  (1)..(21)
<223>  symmetric "ideal" Lac operator as described in Bell, C.E. &
       Lewis, M., Current Opinion in Structural Biology (2001) 11:19-25

<400>  4
gaattgtgag cgctcacaat t                                                21


<210>  5
<211>  17
<212>  DNA
<213>  Escherichia coli


<220>
<221>  misc_feature
<222>  (1)..(17)
<223>  purF operator sequence according to Huffman J.F. et al.
       Biochemistry (2002) 41:511-520

<400>  5
tacgcaaacg ttttctt                                                     17


<210>  6
<211>  16
<212>  DNA
<213>  Escherichia coli


<220>
<221>  misc_feature

<222>  (1)..(16)
<223>  purF operator sequence according to He, B. et al. Journal of
       Bacteriology (1992) 174:130-136

<400>  6
acgcaatcgg ttacct                                                    16


<210>  7
<211>  19
<212>  DNA
<213>  Saccharomyces cerevisiae


<220>
<221>  misc_feature
<222>  (1)..(19)
<223>  Sequence according to FIG. 3 in Liang, S.D., et al. Mol Cell Biol
       (1996) 16:3773-3780

<400>  7
tccggttgac tgtcaaccg                                                 19


<210>  8
<211>  19
<212>  DNA
<213>  Saccharomyces cerevisiae


<220>
<221>  misc_feature
<222>  (1)..(19)
<223>  Sequence according to FIG. 3 in Liang, S.D., et al. Mol Cell Biol
       (1996) 16:3773-3780

<400>  8
tccggtcgac tgtcgaccg                                                 19


<210>  9
<211>  19
<212>  DNA
<213>  Saccharomyces cerevisiae


<220>
<221>  misc_feature
<222>  (1)..(19)
<223>  Sequence according to FIG. 3 in Liang, S.D., et al. Mol Cell Biol
       (1996) 16:3773-3780

<400>  9
tccggtagac tgtctaccg                                                 19

<210> 10
<211> 19
<212> DNA
<213> Saccharomyces cerevisiae

<220>
<221> misc_feature
<222> (1)..(19)
<223> Sequence according to FIG. 3 in Liang, S.D., et al. Mol Cell Biol
      (1996) 16:3773-3780

<400> 10
tccggtgtac tgtacaccg                                                    19

<210> 11
<211> 19
<212> DNA
<213> Saccharomyces cerevisiae

<220>
<221> misc_feature
<222> (1)..(19)
<223> Sequence according to FIG. 3 in Liang, S.D., et al. Mol Cell Biol
      (1996) 16:3773-3780

<400> 11
tccggtgcac tgtgcaccg                                                    19

<210> 12
<211> 1065
<212> DNA
<213> Artificial

<220>
<223> GFP (green fluorescent protein) expression construct including
      the annealing sites for the primers lacOs, lacOr, NlacOs, NlacOr.

<400> 12
atgcgtccgg cgtagaggat cgagatctcg atcccgcgaa attaatacga ctcactatag      60

ggagaccaca acggtttccc tctagaaata attttgttta actttaagaa ggagatatac     120

catgactaaa ggtgaagaac ttttcactgg agttgtccca attcttgttg aattagatgg     180

tgatgttaat gggcacaaat tttctgtcag tggagagggt gaaggtgatg caacatacgg     240

aaaacttacc cttaaattta tttgcactac tggaaaacta cctgttccat ggccaacact     300

tgtcactact ttctcttatg gtgttcaatg cttttcaaga tacccggatc atatgaaacg     360

gcatgacttt ttcaagagtg ccatgcccga aggttatgta caggaaagaa ctatattttt 420

caaagatgac gggaactaca agacacgtgc tgaagtcaag tttgaaggtg atacccttgt 480

taatagaatc gagttaaaag gtattgattt taaagaagat ggaaacattc ttggacacaa 540

attggaatac aactataact cacacaatgt atacatcatg gcagacaaac aaaagaatgg 600

aatcaaagtt aacttcaaaa ttagacacaa cattgaagat ggaagcgttc aactagcaga 660

ccattatcaa caaaatactc caattggcga tggccctgtc cttttaccag acaaccatta 720

cctgtccaca caatctgccc tttcgaaaga tcccaacgaa aagagagacc acatggtcct 780

tcttgagttt gtaacagctg ctgggattac acatggcgtg gatgaactgt accaacccgg 840

ggggggttct catcatcatc atcatcatta ataaaagggc gaattccagc acactggcgg 900

ccgttactag tggatccggc tgctaacaaa gcccgaaagg aagctgagtt ggctgctgcc 960

accgctgagc aataactagc ataacccctt ggggcctcta aacgggtctt gaggggtttt 1020

ttgctgaaag gaggaactat atccggatat ccacaggacg ggtgt 1065

<210> 13
<211> 1080
<212> DNA
<213> Escherichia coli

<220>
<221> misc_feature
<223> LacI coding sequence

<400> 13
gtgaaaccag taacgttata cgatgtcgca gagtatgccg gtgtctctta tcagaccgtt 60

tcccgcgtgg tgaaccaggc cagccacgtt tctgcgaaaa cgcgggaaaa agtggaagcg 120

gcgatggcgg agctgaatta cattcccaac cgcgtggcac aacaactggc gggcaaacag 180

tcgttgctga ttggcgttgc cacctccagt ctggccctgc acgcgccgtc gcaaattgtc 240

gcggcgatta aatctcgcgc cgatcaactg ggtgccagcg tggtggtgtc gatggtagaa 300

cgaagcggcg tcgaagcctg taaagcggcg gtgcacaatc ttctcgcgca acgcgtcagt 360

gggctgatca ttaactatcc gctggatgac caggatgcca ttgctgtgga agctgcctgc 420

actaatgttc cggcgttatt tcttgatgtc tctgaccaga cacccatcaa cagtattatt 480

ttctcccatg aagacggtac gcgactgggc gtggagcatc tggtcgcatt gggtcaccag 540

caaatcgcgc tgttagcggg cccattaagt tctgtctcgg cgcgtctgcg tctggctggc 600

tggcataaat atctcactcg caatcaaatt cagccgatag cggaacggga aggcgactgg 660

agtgccatgt ccggttttca acaaaccatg caaatgctga atgagggcat cgttcccact 720

gcgatgctgg ttgccaacga tcagatggcg ctgggcgcaa tgcgcgccat taccgagtcc 780

gggctgcgcg ttggtgcgga tatctcggta gtgggatacg acgataccga agacagctca 840

tgttatatcc cgccgttaac caccatcaaa caggattttc gcctgctggg gcaaaccagc 900

gtggaccgct tgctgcaact ctctcagggc caggcggtga agggcaatca gctgttgccc 960

gtctcactgg tgaaaagaaa aaccaccctg gcgcccaata cgcaaaccgc ctctccccgc 1020

gcgttggccg attcattaat gcagctggca cgacaggttt cccgactgga aagcgggcag 1080

<210> 14
<211> 52
<212> DNA
<213> Artificial

<220>
<223> lacOs primer

<400> 14
tgttgtgtgg aattgtgagc ggataacaat tcacacccgt cctgtggata tc 52

<210> 15
<211> 52
<212> DNA
<213> Artificial

<220>
<223> lacOr primer

<400> 15
tgttgtgtgg aattgtgagc ggataacaat tatgcgtccg gcgtagagga tc 52

<210> 16
<211> 31
<212> DNA
<213> Artificial

<220>
<223> NlacOs primer

<400> 16
tgttgtgtgg cacacccgtc ctgtggatat c 31

<210> 17

<211>  31
<212>  DNA
<213>  Artificial

<220>
<223>  NlacOr primer

<400>  17
tgttgtgtgg atgcgtccgg cgtagaggat c                                    31